(19) Europäisches Patentamt
European Patent Office
Office européen des brevets

(11) EP 1 578 985 B1

(12) EUROPEAN PATENT SPECIFICATION

(45) Date of publication and mention of the grant of the patent:
**31.10.2007 Bulletin 2007/44**

(21) Application number: **02790638.7**

(22) Date of filing: **31.12.2002**

(51) Int Cl.:
***C12Q 1/00*** *(2006.01)*

(86) International application number:
**PCT/IB2002/005679**

(87) International publication number:
**WO 2004/058993 (15.07.2004 Gazette 2004/29)**

(54) **LACTATE BIOSENSING STRIP**

LACTATE BIODETEKTION STRIP

BANDE DE BIODETECTION DE LACTATE

(84) Designated Contracting States:
**AT BE BG CH CY CZ DE DK EE ES FI FR GB GR IE IT LI LU MC NL PT SE SI SK TR**

(43) Date of publication of application:
**28.09.2005 Bulletin 2005/39**

(73) Proprietor: **COUNCIL OF SCIENTIFIC AND INDUSTRIAL RESEARCH**
**New Delhi 110 001 (IN)**

(72) Inventors:
- **PANDEY,Manoj, Kumar,National Physical Laboratory**
  **New Delhi 110 012 (IN)**
- **CHAUBEY,Asha,National Physical Laboratory**
  **New Delhi 110 012 (IN)**
- **PANDE,Krishan,Kant,National Physical Laboratory**
  **New Delhi 110 012 (IN)**
- **SHARMA,Rajendra,Kumar,National Physical Laboratory**
  **New Delhi 110 012 (IN)**
- **SAINI,Krishan,Kumar,National Physical Laboratory**
  **New Delhi 110 012 (IN)**
- **MALHOTRA,Bansi,Dhar,National Physical Laboratory**
  **New Delhi 110 012 (IN)**
- **RAJESH,National Physical Laboratory**
  **New Delhi 110 012 (IN)**

(74) Representative: **Mattsson, Niklas**
**Awapatent AB**
**P.O. Box 45086**
**104 30 Stockholm (SE)**

(56) References cited:
**WO-A-99/19507**
**US-A- 5 707 502**
**US-A- 5 820 551**

- **CHAUBEY A ET AL: "Immobilization of lactate dehydrogenase on electrochemically prepared polypyrrole-polyvinylsulphonate composite films for application to lactate biosensors" ELECTROCHIMICA ACTA, ELSEVIER SCIENCE PUBLISHERS, BARKING, GB, vol. 46, no. 5, 1 January 2001 (2001-01-01), pages 723-729, XP004225124 ISSN: 0013-4686**
- **GERARD MANJU ET AL: "Immobilization of lactate dehydrogenase on electrochemically prepared polyaniline films." ELECTROANALYSIS, vol. 11, no. 6, May 1999 (1999-05), pages 450-452, XP009015496 ISSN: 1040-0397**
- **GERARD MANJU ET AL: "Application of conducting polymers to biosensors." BIOSENSORS & BIOELECTRONICS, vol. 17, no. 5, May 2002 (2002-05), pages 345-359, XP002250769 May, 2002 ISSN: 0956-5663**
- **CHAUBEY ASHA ET AL: "Immobilization of lactate dehydrogenase on tetraethylorthosilicate-de rived sol-gel films for application to lactate biosensor." APPLIED BIOCHEMISTRY AND BIOTECHNOLOGY, vol. 96, no. 1-3, October 2001 (2001-10), pages 293-301, XP009015497 ISSN: 0273-2289**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

## Description

### Field of the invention

The invention relates to a lactate biosensing strip for the measurement of lactate solution.

### Background of the invention

Physicians rely on personal examination and clinical laboratory results to determine the presence and concentration of biological analytes in critical care patients. Clinical laboratories offer a wide range of automated systems for high-volume testing and analytical support in a well controlled, high quality environment. However, clinical laboratories can not provide the immediate results needed to properly treat trauma and multi organ dysfunction/failure patients.

To meet the clinical need for immediate test results, several technologies are emerging for testing using reliable, automated analyzers at the patient's bedside including electrochemical biosensors, optical fluorescence sensors, paramagnetic particles for coagulation test systems, and micromachined devices for both chemical and immunochemical testing. These technologies have allowed multi-analyte chemistry panels to be performed rapidly and have addressed previous obstacles such as calibration of test devices.

These tests can be classified as: 1) in vitro, which is performed at the bedside; 2) ex vivo or para vivo, which is performed at wrist-side; and 3) in vivo, which is performed inside the patient. Such tests offer indirect cost efficiencies and savings such as reduced labor costs, decreased blood identification and transport errors, and reduced patient complications.

In vitro or bedside devices are used typically in several departments of the hospital including intensive care units; operating rooms; emergency departments (ER); interventional departments; general patient care departments; and outpatient surgery and ambulatory care units. In vitro diagnostic tests offer a wide range of diagnostic tests, similar to the clinical laboratory. In vitro diagnostic test systems typically are not connected on-line to the patient and require an operator for blood sampling.

Key categories of diagnostic test in the diagnostic market include arterial blood gases, blood chemistries, blood glucose, coagulation, drugs-of abuse testing, hemoglobin, hematocrit, infectious diseases, and therapeutic drug monitoring. Other categories include cancer markers, cardiac markers, cholesterol detection, immunodiagnostics, infectious disease detection, lactate, and thrombolytic monitoring.

Ex vivo diagnostics use external sensors for on-line real-time testing with little to no blood loss. Typically, sampled blood flows through a closed system to minimize blood contact. Ex vivo systems minimize problems associated with in vivo sensors, including clotting, inaccuracy, calibration drift, and an inability to recalibrate once in the patient. U.S. Pat. No. 5,505,828 discloses an exemplary ex vivo system.

In vivo diagnostics offer considerable potential in the treatment of most critical and unstable patients. Although many companies are developing in vivo sensors, technical hurdles have thus far kept in vivo sensors from common commercial use.

Ex vivo and in vivo diagnostics, since they are on-line systems, can reduce quality control and information integration errors that occur with clinical or in vitro tests. Quality control errors are commonly due to operator errors, not instrument errors or device failures. Exemplary errors include inappropriate specimen volume, inaccurate calibration, use of deteriorated test strips, inadequate validation, insufficient instrument maintenance, bad timing of the test procedure, and use of the wrong materials. Clinical information system integration allows test data collected at the bedside to be put directly into the patient record. This improves the efficiency of the patient management process, allowing the integration of the laboratory's information system and clinical information systems, providing a "seamless" flow of all types of patient information..

Lactate is the byproduct of carbohydrate metabolism and product of glycolysis (pyrovate) is converted into lactate under an aerobic condition i.e. deficiency of oxygen in cells. Lactate estimations are therefore important in respiratory disorder, heart ailment, labor deseases etc. normal concentration of lactate in human blood is in the range of 1.2 to 2.7mM.

Procedure for lactate determination for example, has employed a variety of chemical and physical technique. Traditional assay involves chemical treatment of lactate in human blood and thereby converting it into colour products which can be measured spectrophotometrically, the methods consists in reacting the blood under test with enzyme namely lactate dehydrogenise (LDH). In such process absorbance at 340nm is measured due to the NADH formation, it becomes a measurement of lactate originally present in blood.

US Patent 6,117,290 discloses an on-line lactate sensor arrangement. The sensor arrangement includes a lactate sensor, a catheter for withdrawing a test sample, and a first fluid flow line provided fluid communication between the lactate sensor and the catheter. The sensor arrangement also includes a source of sensor calibration and anticoagulant solution, and second fluid flow line providing fluid communication between the source of sensor calibration and

anticoagulant solution and the lactate sensor.

In practice there are some difficulties in adopting such a detection procedure for use with blood sample. The disadvantage of such methods, include, lack of specificity, difficulty of standardization, requirement of large amount of blood and use of unstable and corrosive regents. Such methods also involve optical detection and are therefore expensive and time consuming. Additionally, the samples must be prepared. Another disadvantage is that the measurement of lactate level by prior art methods need to be done in laboratory by qualified personnel.

Asha Chaubey et al disclose in Electrochimica Acta Vol 46, 723 - 729 (2000) the immobilization of lactate dehydrogenase on electrochemically prepared polypyrrole polyvinyl sulphonate composite films. The response time reported is about 40 seconds and a shelf life of about 2 weeks under refrigerated conditions. In another disclosure (Asha Chaubey et al, Analyticla Chimica Acta Vol 49, 98 - 103, 2000), the immobilization of lactate dehydrogenase on conducting polyaniline films is disclosed. The linearity of response is shown from 0.1mM to 1mM lactate concentration with a shelf life of about 3 weeks under refrigerated conditions. It is preferable to obtain sensors with longer shelf life and shorter response time.

US 5,820,551 discloses a sensor system for detecting a current representative of a compound in a liquid mixture. The system features a strip comprising an active electrode and a reference electrode. The active electrode is preferably formed of carbon and it further comprises an enzyme and a mediator compound.

US 5,707,502 discloses a sensor for measuring for measuring the concentration of an analyte in a solution. The sensor comprises a substrate layer, an electrode layer, an immobilized enzyme layer and a hydrophobic layer. US 5,707,502 does not disclose the use of an electron mediator for transferring electrodes from the enzyme catalyzed reaction to the electrode layer.

WO 99/19507 discloses a biosensor based on an enzymatic reaction, wherein the enzyme layer, which together with a conductive element forms an electrode, comprises an enzyme (such as glucose dehydrogenase), a nicotinamide cofactor (such as NAD), and a mediator (such as 1,10-phenanthroline quinone) along with other components (such as binders, fillers and stabilizers).

Accordingly, it is important to provide a lactate biosensing strip that can overcome the disadvantages of the prior art without losing out on efficiency and accuracy of measurement.

**Objects of the invention**

The main object of this invention is to provide a novel lactate biosensing strip for the measurement of lactate in aqueous medium.

It is another object of the invention to provide a lactate biosensing strip which performs rapidly and accurately the estimation of lactate in an aqueous medium.

It is yet another object of the invention to provide a lactate biosensing strip which is low cost and is capable of being used by even non-medical persons.

A further object of this invention is an assay, which can be performed without the need for elaborate preliminary treatment of blood sample.

Another object of this invention is to provide a lactate biosensing strip, which has a high activity of 75%.

Still another object of this invention is to provide a lactate-sensing strip, which is capable for providing a reading at site.

**Summary of the invention**

Lactate biosensing strips have many advantages over traditional methods, such as fast response, small size convenience, specificity of response, lack of need of any sample preparation, low cost and high sensitivity of measurement. The main advantage of this sensor over the traditional method is sample operation it can be done by ordinary person.

The present invention provides a lactate biosensing strip for use in the assay of lactate in a sample, said sensor comprising a dry strip sensor of an electrically conducting material having at least:

i. an external surface.
ii. a screen printed reference electrode and
iii. a screen-printed working electrode.

Accordingly, the present invention provides a A lactate biosensing strip comprising

i. an electrically insulated base support (1),
ii. a pair of first and second silver layers deposited thereon (2) separated by an appropriate space between the two said layers,

iii. a pair of graphite layers, each one of said pair of graphite layers being deposited on one respective silver layer and being electrically connected to said respective silver layer (2),
iv. the first silver layer being covered fully by the respective graphite layer,
v. the second silver layer being covered partly in the middle thereof with the respective graphite layer after leaving the connecting terminal and working zone area uncovered,
vi. the uncovered working zone of said second silver electrode layer being deposited with silver chloride (4),
vii. lactate oxidase being deposited with a mediator on the working zone of graphite layer covering the first silver layer (5),
viii. the said silver/silver chloride layer forming reference electrode (4) and enzyme with mediator layer forming working electrode (5) being supported on said support (1),
ix. the working zone of reference electrode (4) and working electrode (5) being covered with a hydrophilic membrane.

In one embodiment of the invention, the electrically insulated base support used is made of polyvinyl chloride.
In one embodiment of the invention the distance between the silver layers is in the range of 0.5 to 1mm
In another embodiment of the invention, the thickness of each silver layer is in the range of 15 to 25 microns.
In another embodiment of the invention, the electron mediator layer comprises a layer of potassium ferricyanide or ferrocene.
In another embodiment of the invention, the hydrophilic membrane is made of nylon or polyester.
In another embodiment of the invention, the working zone are of electrode is a target area used for dispensing the analyte sample
In another embodiment of the invention, the connecting terminal zone area of electrode is an area used for the connectivity of electrode to an electrometer
The lactate biosensing strip of the invention shows an activity of 75% and a response time for lactate detection is in the range of 30 to 40 seconds. The shelf life of the strip of the invention is about 4 months under refrigerated conditions. Under ambient conditions (25 to 30°C) the shelf life of the biosensing strip is seen to be about 2 months. The strip of the invention is disposable.

**Brief description of the accompanying drawings**

Figure 1 is a schematic representation of the biosensing strip of the invention.
Figure 2 is the response curve of the lactate biosensing strip of the invention for standard lactate test samples.
Figure 3 shows the calibration curve for the sensor against standard lactate test samples prepared in a laboratory.
Figure 4 shows the shelf life stability characteristics of the lactate strip of the invention.

**Detailed description of the invention**

As shown in figure 1, the invention comprises an electrically insulated base support (1) for supporting an electrode assembly (2), (3), (4) and (5). The electrode assembly comprises two electrode systems, a working electrode system (2), (3) and (5) consisting of a silver layer with a graphite layer deposited thereon and an enzyme and mediator layer adsorbed in the inorganic matrix. The other electrode assembly comprises a reference electrode comprising a silver layer partly deposited with a graphite layer and a silver/silver chloride layer thereon. Figure 1 shows the PVC sheet (i) which comprises the supporting substrate for the electrode. Conducting silver tracking (ii) is the screen-printed conducting graphite layer onto the surface of conducting silver tracking (iii) for the connection of the sensor to read out apparatus. The target area consists of the working electrode (iv) and the reference electrode (v) applies to the end of tracking by screen-printing. An insulated layer is applied over the printed electrode to give them protection; the mass can be coated with one or more legends. The conducting graphite track (ii) does not extend to the complete length of the silver track and the reference electrode.
To achieve calibration of the biosensing strip, the strip was used to detect currents when the lactate solutions were used in concentrations of 1 to 8mM. The current measured for each of the concentrations was measured and plotted in Figure 2. In Figure 2, curve (1) is the response curve for 1mM lactate solution, curve (2) is for 2mM solution, curve (3) is for 4mM solution, curve (4) is for 6mM solution and curve (5) is for 8mM solution. This shows that the biosensing strip of the invention can be used to measure lactate in a blood sample if the range lies in the region of 1 to 8 mM in a subject. The sensitivity of the system in terms of the response time to attain a stable current value was determined by analyzing the strip time variation of current. This comprised initiating current measurement from the time fo putting the drop of standard test solution on the strip to the time when the current asymptotically reaches a stable value. It was observed (Figure 3) that the current attains the stable value in 30 to 40 seconds.

Shelf life characteristics were determined by measuring the current due to a known lactate concentration on strips stored for different periods of time. The data is given in Figure 4. In Figure 4, curve (1) is for strips stored under refrigerated conditions (at 4°C) while curve (2) is for strips stored at 25 - 30°C.

The invention also provides a process for producing a lactate sensor strip which comprise in forming a first and second electrode on a substrate by applying a layer of silver for each of said electrodes in said electrode, applying a layer graphite on the handling zone of said second electrode to silver chloride, applying a mediator and enzyme on the graphite layer of the working zone of the first electrode. An outer hydrophilic membrane is applied zone of said first electrode. The silver layers and the graphite layers are preferably applied by the step of screen-printing.

The main feature of this invention is that the sensor is a dry strip sensor. It is found that a similar mix of reagents employed in a wet sensor system did not give good result across a desired range of detectable lactate concentration.

This invention comprises a substrate for supporting an electrode assembly said electrode assembly comprising two electrode systems, one working electrode and another one as a reference electrode supported on said substrate and disposed in a spaced relationship to each other. The lactate sensing strip comprising of a substrate for supporting a first or working electrode and second or reference electrode, said electrode disposed in a spaced relationship to each other.

The first electrode is a working electrode and has a terminal extending in to a working zone through a handling zone. The second electrode is a reference electrode and has a terminal extending in to a working zone through a handling zone. In both cases, the respective terminals are of a material different to the base conducting layer of said first and second electrodes.

Commercially obtained lactate oxidase is mixed in a phosphate buffer, then proper amount of this solution is injected onto a preprinted working electrode. This solution is allowed to dry in allow temperature, followed by

i. printing of conducting tracking
ii. printing of reference electrode
iii. printing of working electrode
iv. fixing of membrane onto electrode.

The working and reference electrode each comprise a base conducting layer of silver material along the handling and working zone. A graphite layer is deposited on the silver layer of the working electrode and extends to the terminal; the graphite layer is applied on the handling zone of the reference electrode and extends to the terminal. Ag/AgCl is deposited on the target area of the reference electrode. Working electrode comprising conducting surface carrying mediator compound and lactate oxidase enzyme. Mediator compound transfer electrodes from the enzyme to the electrode, when such catalytic activity takes place. A hydrophilic membrane must be provided on the working zone of said electrode. It appears that the surfactant serves to break up the lipoprotein complex of blood and lactate is then oxidized to the pyruvate by the lactate oxidase. The mediator compound is electrochemically reduced at the electrode producing a current measurable at the electrode, which current is relative to the activity of the lactate oxidize and hence the amount of lactate present in the sample this current is generated through a serious of coupled reactions

$$\text{L-Lactate+LOD}_{(ox)} \text{ ----------------- Pyruvate +LOD}_{(red)}$$

$$\text{LOD}_{(red)} \text{ +Me}_{(ox)} \text{ ----------------- LOD}_{(ox)} \text{ + Me}_{(re)}$$

The redox mediator is oxidized at the base electrode and the current proportional to the lactate concentration. The current can be measured by any conventional electronic system.

The following examples are given by the way of illustration and therefore should not constitute to limit the scope of the present invention.

**Example 1**

**Preparation of graphite paste with mediator**

100mg of graphite powder and polyvinyl pyrrolidon (binder) was mixed with 0.01M Potassium ferricyanide (mediator) in ethylene glycol monobutyl ether to prepare screen printable working electrode graphite paste.

**Example 2**

**Preparation of Dry strip**

Commercially obtained lactate oxidase solution (2μL) containing 2U of lactate oxidase was physically adsorbed on the mediator mixed graphite electrode strip and was kept over night to dry at 25°C. The dry strip electrode was covered with a hydrophilic nylon membrane. Before the membrane was applied, it was placed in 10% surfactant (Tween 80) solution in distilled water for some time the dried membrane was then fixed over the strip.

**Example 3**

**Preparation of lactate standard lactate solutions**

Stock lactate solution 10 mM was prepared in 0.1M phosphate buffer. Standard solutions of 2 mM, 4 mM, 6 mM and 8 mM were prepared by diluting the stock solution with phosphate buffer.

**Example 4**

**Preparation of enzyme stock solution**

15 mg of enzyme lactate oxidase was dissolved in 100μl of 0.1M phosphate buffer to get the concentration 5U/μ.l to get the working enzyme solution, the stock solution was further diluted to 1U/μl.

**Example 5**

**Immobilization of enzyme on the mediator mixed graphite dry strip**

2 μl of enzyme solution containing 2 U of lactate oxidase was physically adsorbed on the mediator mixed graphite electrode strip and was kept over night to dry at 25°C. The said dry strip electrode was covered by a hydrophilic nylon membrane. Before applying the membrane, it was placed in 10% surfactant (Tween 80) solution in distilled water for some time and then dried membrane was fixed over the strip.

**Example 6**

**Enzyme activity**

Sigma protocol for activity of lactate oxidase was used to estimate the lactate oxidase activity. The basic principle is that lactate oxidase converts 1-lactate to pyruvate and $H_2O_2$. $H_2O_2$ is subsequently converted into a colored dye by peroxidase in the presence of 4-amino antipyrine (4AAP) and dimethylaniline(DMA).

$$\text{L-lactate} + O_2 \xrightarrow{\text{LOD}} \text{Pyruvate} + H_2O_2$$

$$2\,H_2O_2 + \text{4-AAP} + \text{DMA} \xrightarrow{\text{LOD}} \text{Quinonediimine dye} + H_2O$$

In the optimum conditions of temperature= 37°C and pH = 6.5, the dye absorbs at 565 nm at the light path of 1 cm.

The activity of the immobilized enzyme was calculated according to the following formula:

$$U\,cm^{-2} = AV/\varepsilon\,t\,s$$

Where A is the change in absorbance before and after incubation

V is the total volume (3ml)
s is the milimolar extinction coefficient of Quinonediimine dye at 565 nm (35.33)
t is the reaction time (10 min)
s is the surface area of the enzyme electrode

The enzyme activity of immobilized LOD on the working graphite strip was found to be 75%.

**Example7**

**Amperometric Response Studies**

The lactate biosensing strip comprising enzyme(LOD) immobilized on graphite as working electrode and Ag/AgCl reference electrode is connected to the input of the electrometer was polarized at a bias voltage of 0.4V for the measurement of amperometric calibration response to lactate (1-8 mM)(Figure 2). A maximum current of 60 μA was obtained for 8 mM lactate solution above which no significant change in current could be observed. The response time for lactate solution (1-8 mM) was found to be 40 seconds for each concentration of lactate (Figure 3). Results were found to be reproducible to within 5%. Following principle was involved in the amperometric measutrements.

$$\text{Lactate} + \text{LOD (ox)} \longrightarrow \text{Pyruvate} + \text{LOD (red)}$$

$$\text{LOD (red)} + Fe^{3+} \longrightarrow \text{LOD (ox)} + Fe^{2+}$$

$$Fe^{2+} \xrightarrow{0.4V} Fe^{3+}$$

**Advantages of the invention**

1. The lactate biosensing strip provides a quick estimation of lactate in a sample
2. the shelf life of the sample is 4 months under refrigerated conditions.
3. the strip has a linear response in a lactate concentration of 1 to 8 mM.
4. the strip is disposable without causing any environmental hazard.
5. the strip is easily used even by people without any formal medical training.

**Claims**

**1.** A lactate biosensing strip comprising an electrically insulated base support (1), a pair of first and second silver layers deposited thereon (2) separated by an appropriate space between the two said layers, a pair of graphite layers, each one of said pair of graphite layers being deposited on one respective silver layer and being electrically connected to said respective silver layer (2), the first silver layer being covered fully by the respective graphite layer, the second silver layer being covered partly in the middle thereof with the respective graphite layer after leaving the connecting terminal and working zone area uncovered, the uncovered working zone of said second silver electrode layer being deposited with silver chloride (4), lactate oxidase being deposited with a mediator on the working zone of graphite layer covering the first silver layer (5), the said silver/silver chloride layer forming reference electrode (4) and enzyme with mediator layer forming working electrode (5) being supported on said support (1).

**2.** A biosensing strip as claimed in claim 1 wherein the working zone of reference electrode and working electrode are covered with a hydrophilic membrane.

**3.** A biosensing strip as claimed in claim 1 wherein the electrically insulated base support used is made of polyvinyl

chloride.

4. A biosensing strip as claimed in claim 1 wherein the distance between the silver layers is in the range of 0.5 to 1 mm

5. A biosensing strip as claimed in claim 1 wherein the thickness of each silver layer is in the range of 15 to 25 microns.

6. A biosensing strip as claimed in claim 1 wherein the electron mediator used is selected from potassium ferricyanide or ferrocene.

7. A biosensing strip as claimed in claim 2 wherein the hydrophilic membrane used is made of nylon or polyester.

8. A biosensing strip as claimed in claim 1 wherein the working zone of an electrode is a target area used for dispensing the analyte sample.

9. A biosensing strip as claimed in claim 1 wherein the enzyme lactate oxidase activity in the strip is in the range of 70-80 %.

10. A biosensing strip as claimed in claim 1 wherein the response time for lactate detection is in the range of 30 to 40 seconds.

11. A biosensing strip as claimed in claim 1 wherein the amperometric linear response to lactate concentration is in the range of 2-8 mM.

12. A biosensing strip as claimed in claim 1 wherein the shelf life of the biosensing strip is about 4 months under refrigerated conditions.

13. A biosensing strip as claimed in claim 1 wherein the shelf life of the biosensing strip is about 2 months at a temperature in the range of 20-30 °C.

14. A biosensing strip as claimed in claim 1 wherein the strip is disposable.

15. A biosensing strip as claimed in claim 1 wherein the connecting terminal of electrode is an area used for connecting an electrode to an electrometer.

## Patentansprüche

1. Ein Lactat-Biosensor-Streifen, der
eine elektrisch isolierte Basisunterlage umfasst (1),
ein Paar von ersten und zweiten Silberschichten, die darauf abgeschieden sind (2) und getrennt sind durch einen geeigneten Zwischenraum zwischen den beiden Schichten,
ein Paar von Graphitschichten, wobei jede von besagtem Paar von den Graphitschichten auf einer entsprechenden Silberschicht abgeschieden wird und elektrisch verbunden wird mit besagter entsprechender Silberschicht (2),
wobei die erste Silberschicht vollständig durch die entsprechende Graphitschicht bedeckt ist, die zweite Silberschicht teilweise in der Mitte davon mit der entsprechenden Graphitschicht bedeckt ist, nachdem die verbindenden End- und Arbeits-Zonen-Bereiche unbedeckt bleiben,
den unbedeckte Arbeitsbereich von besagter zweiter Silberelektrodenschicht, auf dem Silberchlorid abgeschieden worden ist (4),
Lactatoxidase, die abgeschieden worden ist mit einem Mediator auf dem Arbeitsbereich der Graphitschicht, welche die erste Silberschicht bedeckt (5),
wobei besagte Silber/Silber-Chloridschicht eine Referenzelektrode (4) ausbildet und das Enzym mit der Mediatorschicht die Arbeitselektrode (5) ausbildet, unterstützt auf besagter Unterlage (1).

2. Ein Biosensor-Streifen wie in Anspruch 1 beansprucht, wobei der Arbeitsbereich der Referenzelektrode und der Arbeitselektrode mit einer hydrophilen Membran bedeckt sind.

3. Ein Biosensor-Streifen wie in Anspruch 1 beansprucht, wobei die elektrisch isolierte Basisunterlage, die eingesetzt wird, aus Polyvinylchlorid besteht.

4. Ein Biosensor-Streifen wie in Anspruch 1 beansprucht, wobei der Abstand zwischen den Silberschichten im Bereich von 0,5 bis 1 mm liegt.

5. Ein Biosensor-Streifen wie in Anspruch 1 beansprucht, wobei die Dicke einer jeden Silberschicht im Bereich von 15 bis 25 μm liegt.

6. Ein Biosensor-Streifen wie in Anspruch 1 beansprucht, wobei der Elektronen-Mediator, der eingesetzt wird, ausgewählt wird aus Kaliumferrocyanid oder Ferrocen.

7. Ein Biosensor-Streifen wie in Anspruch 2 beansprucht, wobei die hydrophile Membran, die eingesetzt wird, aus Nylon oder Polyester besteht.

8. Ein Biosensor-Streifen wie in Anspruch 1 beansprucht, wobei der Arbeitsbereich einer Elektrode ein Zielbereich ist, der verwendet wird zum Auftragen der Analytprobe.

9. Ein Biosensor-Streifen wie in Anspruch 1 beansprucht, wobei die Lactatoxidase- Enzym-Aktivität in dem Streifen in der Größenordnung von 70-80% liegt.

10. Ein Biosensor-Streifen wie in Anspruch 1 beansprucht, wobei die Antwortzeit für den Lactatnachweis in der Größenordnung von 30-40 Sekunden liegt.

11. Ein Biosensor-Streifen wie in Anspruch 1 beansprucht, wobei die lineare Amperometer-Antwort auf die Lactatkonzentration in der Größenordnung von 2-8 mM liegt.

12. Ein Biosensor-Streifen wie in Anspruch 1 beansprucht, wobei die Haltbarkeitsdauer des Biosensor-Streifens ungefähr 4 Monate unter Kühlschrankbedingungen ist.

13. Ein Biosensor-Streifen wie in Anspruch 1 beansprucht, wobei die Haltbarkeitsdauer des Biosensor-Streifens ungefähr 2 Monate bei einer Temperatur in der Größenordnung von 20-30° C ist.

14. Ein Biosensor-Streifen wie in Anspruch 1 beansprucht, wobei der Streifen wegwerfbar ist.

15. Ein Biosensor-Streifen wie in Anspruch 1 beansprucht, wobei das verbindende Ende der Elektrode ein Bereich ist, der für das Verbinden einer Elektrode an ein Elektrometer eingesetzt wird.

## Revendications

1. Bande de biodétection de lactate comprenant un support de base isolé électriquement (1), une paire de première et deuxième couches d'argent (2) déposées par dessus, séparées par un espace approprié entre les deux dites couches, une paire de couches de graphite, chacune de ladite paire de couches de graphite étant déposée sur une couche d'argent respective et étant connectée électriquement à ladite couche d'argent respective (2), la première couche d'argent étant couverte entièrement par la couche de graphite respective, la deuxième couche d'argent étant couverte partiellement en son milieu par la couche de graphite respective après avoir laissé la zone de la borne de connexion et la zone de travail découvertes, la zone de travail découverte de ladite deuxième couche d'électrode en argent étant déposée avec du chlorure d'argent (4), la lactate oxydase étant déposée avec un médiateur sur la zone de travail de la couche de graphite couvrant la première couche d'argent (5), ladite couche d'argent/de chlorure d'argent formant l'électrode de référence (4) et l'enzyme avec la couche de médiateur formant l'électrode de travail (5) étant supportées sur ledit support (1).

2. Bande de biodétection selon la revendication 1, dans laquelle la zone de travail de l'électrode de référence et l'électrode de travail sont couvertes par une membrane hydrophile.

3. Bande de biodétection selon la revendication 1, dans laquelle le support de base isolé électriquement utilisé est fabriqué en chlorure de polyvinyle.

4. Bande de biodétection selon la revendication 1, dans laquelle la distance entre les couches d'argent se situe dans la plage de 0,5 à 1 mm.

5. Bande de biodétection selon la revendication 1, dans laquelle l'épaisseur de chaque couche d'argent est située dans la plage de 15 à 25 microns.

6. Bande de biodétection selon la revendication 1, dans laquelle le médiateur électronique utilisé est choisi entre le ferricyanure de potassium et le ferrocène.

7. Bande de biodétection selon la revendication 2, dans laquelle la membrane hydrophile utilisée est faite de nylon ou de polyester.

8. Bande de biodétection selon la revendication 1, dans laquelle la zone de travail d'une électrode est une zone cible utilisée pour répartir l'échantillon d'analyte.

9. Bande de biodétection selon la revendication 1, dans laquelle l'activité enzymatique de la lactate oxydase dans la bande est située dans la plage allant de 70 à 80 %.

10. Bande de biodétection selon la revendication 1, dans laquelle le temps de réponse pour la détection du lactate se situe dans la plage allant de 30 à 40 secondes.

11. Bande de biodétection selon la revendication 1, dans laquelle la réponse linéaire ampérométrique à la concentration en lactate est située dans la plage allant de 2 à 8 mM.

12. Bande de biodétection selon la revendication 1, dans laquelle la durée de vie en pot de la bande de biodétection est d'environ 4 mois dans des conditions réfrigérées.

13. Bande de biodétection selon la revendication 1, dans laquelle la durée de vie en pot de la bande de biodétection est d'environ 2 mois à une température située dans la plage allant de 20 à 30°C.

14. Bande de biodétection selon la revendication 1, dans laquelle la bande est jetable.

15. Bande de biodétection selon la revendication 1, dans laquelle la borne de connexion de l'électrode est une zone utilisée pour connecter une électrode à un électromètre.

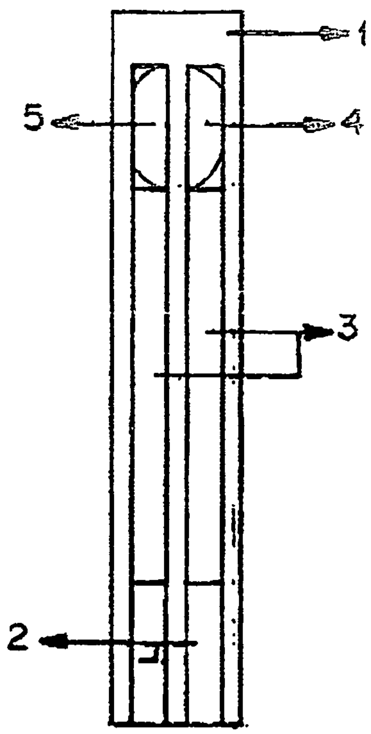


Fig. 1

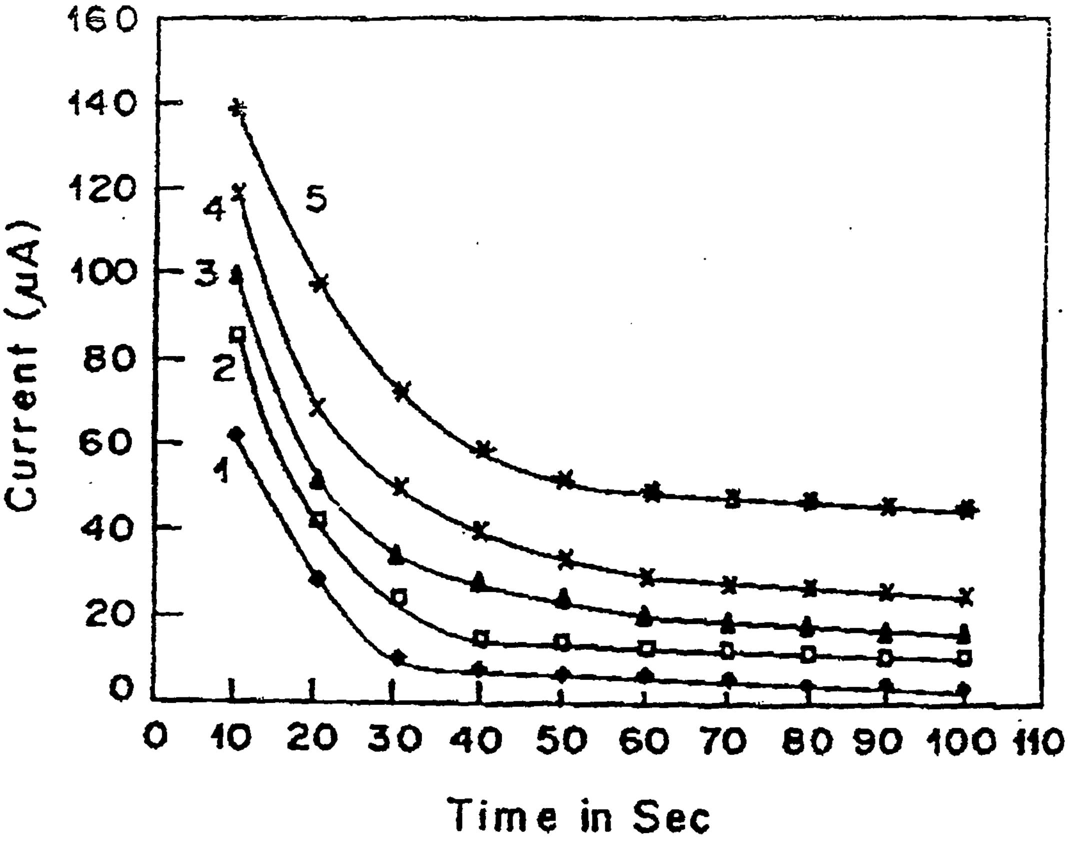
160
140
120
100
80
60
40
20
0
Current (μA)
0 10 20 30 40 50 60 70 80 90 100 110
Time in Sec
1
2
3
4
5

Fig. 2

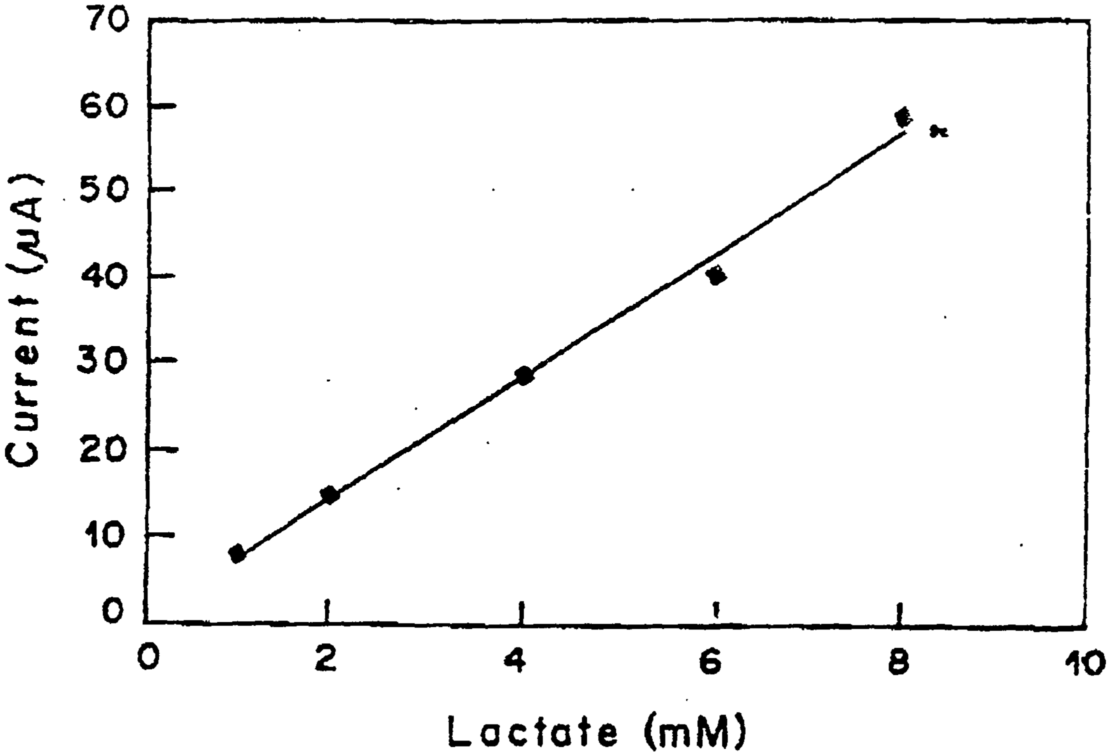

Fig.3

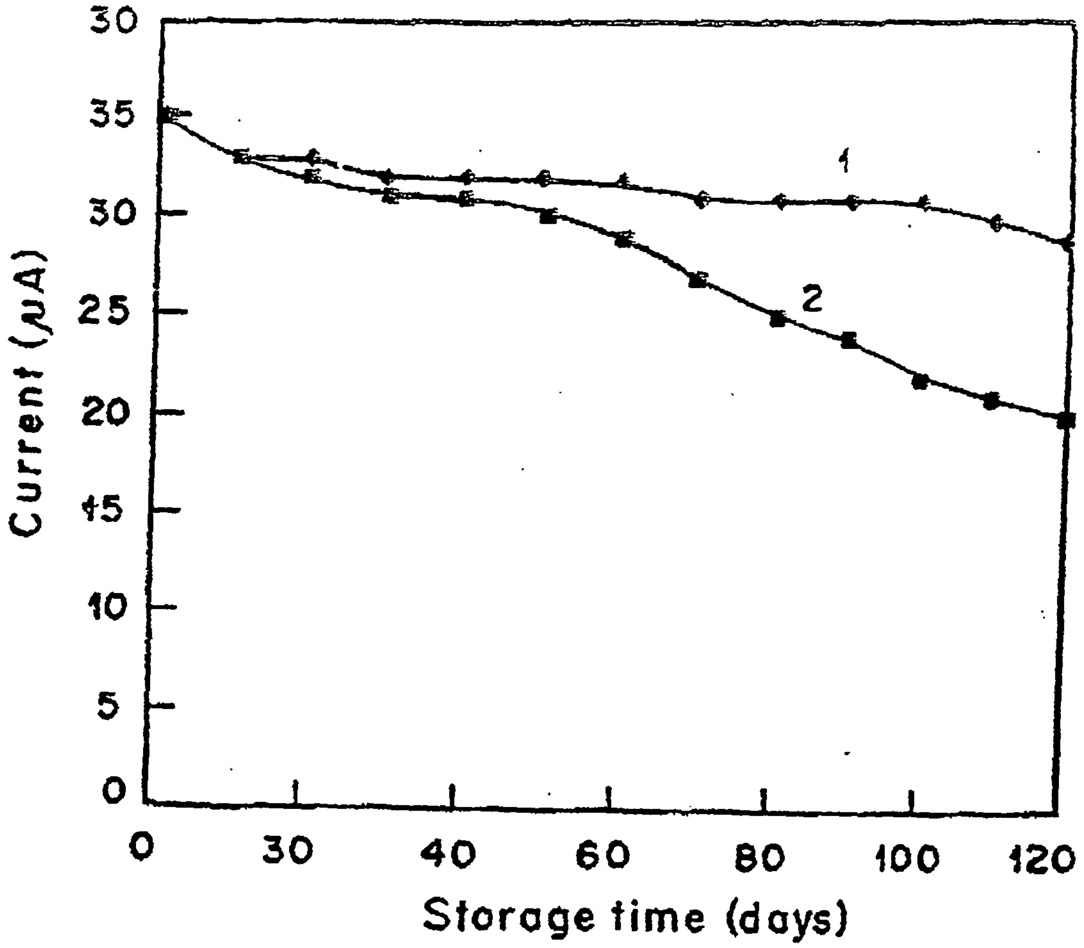
30
35
30
25
20
15
10
5
0
Current (μA)
1
2
0
30
40
60
80
100
120
Storage time (days)

Fig. 4

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 5505828 A
- US 6117290 A
- US 5820551 A
- US 5707502 A
- WO 9919507 A

### Non-patent literature cited in the description


- **ASHA CHAUBEY et al.** *Electrochimica Acta,* 2000, vol. 46, 723-729
- **ASHA CHAUBEY et al.** *Analyticla Chimica Acta,* 2000, vol. 49, 98-103